# EUROPEAN PATENT SPECIFICATION

(11) **EP 1 001 781 B1**
(45) Date of publication and mention of the grant of the patent: **22.02.2006**
(21) Application number: 98937635.5
(22) Date of filing: 31.07.1998
(51) Int. Cl.: A61K 31/54, A61K 31/715, A61P 35/04

(54) **USE OF TAUROLIDINE OR TAURULTAM FOR THE MANUFACTURE OF A MEDICAMENT FOR THE PREVENTION OF METASTASES**
VERWENDUNG VON TAUROLIDIN ODER TAURULTAM ZUR HERSTELLUNG EINES ARZNEIMITTELS ZUR VORBEUGUNG VON METASTASEN
UTILISATION DE TAUROLIDINE OU DE TAURULTANE POUR PRODUIRE UN MEDICAMENT PERMETTANT DE PREVENIR DES METASTASES

(30) Priority: 31.07.1997 GB 9716219
(43) Date of publication of application: 24.05.2000
(73) Proprietor: Ed Geistlich Söhne AG Für Chemische Industrie, 6110 Wolhusen (CH)
(72) Inventor: PFIRRMANN, Rolf, CH-6000 Lucerne 15 (CH)
(74) Representative: Pett, Christopher Phineas
(86) International application number: PCT/GB1998/002311
(87) International publication number: WO 1999/006114

(56) References cited:
- WO-A-92/00743
- WO-A-95/18638
- WO-A-95/30423
- DE-A- 19 606 897
- C.A.JACOBI ET AL.: "Peritoneale instillation von Taurolidin und Heparin zur Verhinderung von intraperitonealem Tumorwachstum und Trokarmetastasen bei laparoskopischen Operationen im Rattenmodell" LANGENBECKS ARCH CHIR, vol. 382, no. 4 suppl 1, 25 July 1997, pages S31-S36, XP002083832
- DATABASE WPI Section Ch, Week 8607 Derwent Publications Ltd., London, GB; Class A96, AN 86-045778 XP002091627 & JP 61 000017 A (SEIKAGAKU KOGYO CO LTD) , 6 January 1986

## Description

This invention relates to prevention or reduction of metastases. More particularly it relates to prevention or reduction of metastatic growth following laparoscopic surgery, especially laparoscopic surgery to combat abdominal malignancies.

Malignant tumours within the body, particularly the abdomen, are frequently removed surgically. The exploration and excision of tumours by major invasive surgery has been used for many years but, more recently, minimal invasive surgery has increasingly been used.

A wide range of indications of malignant tumours exist for which invasive surgery, such as laparotomy or laparoscopy, may used. These include, but are not restricted to, the following: oesophagus carcinoma (plaster cell carcinoma, adenocarcinoma) and cardiacarcinoma; malignant degenerative ulcus; carcinoma of the stomach, antrum or corpus, malign adenoma of island cells, re-section or total gastrectomy; carcinoma of the gall duct or distal choledochus; carcinoma of the pancreas head, papilla, corpus or cauda; carcinoma of the small or large intestinal tract, sarcoma; colon malignancy: adeno carcinoma, lymphoma, malign carcinoid, melanoma, fibrosarcoma; carcinoma of the rectum; ovarial carcinoma; mamma carcinoma; and prostate carcinoma.

The use of minimal invasive surgery has brought with it a reduced mortality and a reduced post-operative infection rate. Classic open abdominal surgery such as laparotomy may require less operation time than minimal invasive surgery, but involves long post-operative convalescence and a greater risk of infection, e.g. sepsis. One reason why minimal invasive laparoscopies are on the increase is the drastically reduced amount of time that the patient needs to spend recuperating both in hospital and at home. Laparoscopy also has the advantage that there is a significant reduction in wound scars and in post-operative complications associated with wound healing.

A wide range of laparoscopic procedures are in general use, including laparoscopic cholecystectomy, laparoscopic fundoplicatio (anti-reflex surgery for gastro-oesophageal disease), laparoscopic treatment of para-oesophageal hernia, laparoscopic treatment of abdominal cysts (e.g. liver cysts removed by cystectomy), laparoscopic liver re-section, laparoscopic appendectomy, laparoscopic treatment of intestinal obstruction (e.g. incarcerated hernias, colon obstruction and massively dilated small bowel obstruction), laparoscopic colo-rectal surgery (e.g. ileosacral resection, hemicolectomy, sigma-resection, rectum prolapse and rectum amputation), laparoscopic adhesiolysis, emergency laparoscopy (explorative diagnosis), differential diagnosis of appendicitis, acute abdomen, ileus, abdominal trauma, and oncological queries (e.g. to determine whether or not carcinoma is operable).

One aspect of minimally invasive laparoscopies which gives rise to concern, particularly when these are used to combat abdominal malignancies, is the extent to which metastatic growth has been observed. It is now recognised that manipulation of a malignancy can result in a disturbance and release of malignant cells which can then travel to other locations where, if they adhere and start growing, they may form metastases with predictably unfortunate results. This risk is lower during a classic open laparotomy, for example, where the whole tumour may be carefully excised and removed without transferring any cells to other parts of the abdomen. In a minimally invasive laparoscopy using a trocar, however, this may not be possible and disturbance of the tumour and its contact with adjacent tissues whilst being removed are inevitable. It has been found that "trocar metastases" are often a result of minimally invasive abdominal surgical procedures, e.g. laparoscopic surgery.

One reason for the frequent observation of metastases following laparoscopic intervention is believed to reside in the use of trocar tubes or sleeves, the diameters of which may range from 5 to 20 mm. These can either result in damage to malignant tissues or may otherwise come into contact with cell- , rich exudate which then drips from the trocar sleeve into the abdominal cavity, thereby initiating metastases. To effect the removal of resected organs or pieces from the abdomen, particularly when removing inflamed resections or neoplastic tissue, a "rescue" bag may be introduced *via* the trocar sleeve in an attempt to prevent contamination of the abdominal cavity by resected neoplastic cells or cell threads of the primary tumour.

It is known from WO-A-9200743 that the methylol transfer agents taurolidine and taurultam may be used in treatment or prophylaxis of tumours and may prevent the spread of metastases, for example following surgical removal of tumours.

Jacobi *et al*. in Langenbecks Arch. Chir. (1997) 382 [Suppl. 1]: S31-36 report that intraperitoneal instillation of taurolidine, optionally in combination with heparin, causes a significant decrease in both intraperitoneal tumour growth and trocar metastases in laparoscopic surgery in a rat model.

We have found that particularly effective prevention or reduction in metastatic growth following laparoscopy may be achieved if solutions of taurolidine and/or taurultam are administered to a patient both by passage through a trocar tube or sleeve and by intravenous infusion, each of these forms of administration being performed prior to, during or after the minimally invasive laparoscopic surgical procedure.

The invention accordingly provides use of a solution comprising taurolidine, taurultam or a mixture thereof in the manufacture of a medicament for preventing or reducing metastatic growth following the use of trocars during laparoscopic surgery, characterised in that said medicament is manufactured for administration (i) in a first portion through a trocar tube or sleeve prior to, during or after said sugery and (ii) in a second portion by intravenous infusion prior to, during or after said surgery.

A preferred solution for use in accordance with the invention will contain from 0.5 to 3% by weight of taurolidine, or from 2 to 3% by weight taurultam, depending on the solubility of the compound. Solutions containing from 0.5 to 1.0% or 2.0% taurolidine are preferred.

The solutions will generally be made up in sterile pyrogen-free water and may also contain, for example, inorganic or other salts or other components to render them isotonic. Parenterally acceptable polyols may, for example, also be present since these have been observed to increase the overall intravenous tolerance of taurolidine. Suitable polyols include carbohydrates, e.g. hexoses such as glucose and fructose (or mixtures of these such as invert sugar), pentoses such as xylose or polysaccharides such as dextran or hydrolysed starch; glycerol and sugar alcohols such as sorbitol, mannitol or xylitol.

The concentration of the polyol can usefully be in the range 3-40% by weight. In the case of glucose, the concentration may be in the range 10-30% by weight, preferably 20%.

The solutions may also contain polyvinylpyrrolidone (PVP). This may be incorporated into the solutions at a concentration of, for example, from 4 to 7% by weight. A solution containing 5% PVP is preferred. This assists in solubilising the taurolidine and/or taurultam and contributes also to the oncotic pressure of the solution. The molecular weight of the PVP should not be greater than 30,000 and is preferably less than 10,000, for example between 7000 and 9000. Kollidone 17 as sold by BASF is relatively quickly resorbed and excreted renally.

The exact mode of action of taurolidine and/or taurultam in preventing metastatic growth under these circumstances is still not known. Without wishing to be bound by theoretical considerations, we believe that taurolidine and taurultam are capable of altering the protein surface structure of adhesion molecules (receptors) such as I-P-selectin and fibronectin. It is believed that over-expression of such molecules, and also molecules such as integrin, vitronectin and laminin, is the principal cause of metastatic development, since these molecules are believed to provide the malignant cells with the ability to migrate and adhere to other cell surfaces and endothelium, in particular to vascular endothelium. The malignant cells then become sedentary, allowing themselves to grow and further develop (metastasis). Once developed, such cells are able to reach every organ through either the haematogenic or lymphatic channels (formation of metastases).

It is believed that taurolidine and taurultam modify the surface structure of malignant cells in such a way that over-expression of adhesion molecules is reduced. As a result, adhesion of the malignant cells to other cell surfaces and endothelium, e.g. to vascular endothelium, is reduced or does not occur before the cell itself dies. Taurolidine and taurultam are not believed to have any direct cytotoxic effect on the malignant cells. They are, however, believed to prevent high levels of cytokines such as IL-1β in peritoneal fluid, which in turn prevents tumour cell proliferation and adhesions. Taurolidine and taurultam are thus being used essentially prophylactically in accordance with the invention.

In general, in preparation for laparoscopy the abdominal wall is lifted. This may be achieved either by insufflation (pneumoperitoneum) or mechanically. Special instruments are required to raise the abdominal wall without causing damage to the intestinal loops. A Veres needle having an opening on one side through which a gas may enter the abdominal cavity is generally used for preparation of a pneumoperitoneum. Gases conventionally used for insufflation include N₂O, CO₂ and helium, and may be introduced into the abdominal cavity at a rate of up to 1 litre/min. Depending on the patient's body size and tissue tension, between 3 and 5 litres of CO₂ gas may be required. For diagnostic laparoscopy under local anaesthetic, N₂O is preferred since, unlike CO_{2,} it does not irritate the peritoneum. Whilst not wishing to be bound by theory, it is believed that this irritation could be one of the reasons for the more frequent appearance of metastases observed when using CO₂.

A metal suspension bar is conveniently used to lift the abdominal wall mechanically. Once inserted into the abdomen, special hooks are attached to the suspension bar and the abdomen is then raised using a chain and suspension scale.

According to the type of laparoscopic procedure, from 100-1000 ml, preferably from 100-250 ml, of a 2%, 1% or 0.5% taurolidine solution can be instilled at body temperature via a trocar tube or sleeve and allowed to remain in the abdominal cavity after the end of the operative procedure, and before extraction of the gas used to generate the pneumoperitoneum and final removal of the trocar.

For the prophylaxis of trocar metastases, a 2% Taurolin, 0.5% Taurolin-Ringer or 2-3% taurultam solution may be used. Conveniently, the abdomen is rinsed with such a solution using a rinse-suction tube introduced *via* a trocar tube or sleeve. A 5 or 10 litre rinse bag is filled with the desired rinse solution (isotonic saline or Ringer solution) and hung at a height of approx. 2 m. 1-2 litres of rinse solution are then introduced through the rinse-suction tube. Following a short contact time (sufficient to ensure that the intestinal loops are completely covered by the rinse solution) the solution is then suctioned off. In cases of severe inflammation, the rinsing solution will appear opaque such that abdominal visibility using the optic and camera is poor. In such cases, this rinsing procedure must be repeated until the liquid in the abdomen is clear and translucent.

When the rinsing procedure is complete and the solution is clear, the rinse bag is then filled with 250 ml 2% Taurolin (pre-warmed to 37°C) which is allowed to flow into the abdominal cavity. Finally, a drain is inserted before final removal of the trocar(s) and closure of the abdomen. In severe cases, e.g. severe peritonitis, it is possible to instill (and in some cases to leave) up to 1000 ml Taurolin 2% solution within the abdominal cavity. In place of a 2% Taurolin solution, 1-1.5 litres Taurolin-Ringer 0.5% solution or a 2-3% taurultam solution may be used.

Alternatively, a 2% Taurolin solution may be instilled and suctioned off *via* a trocar tube or sleeve using a pressure-rinse apparatus. Another variation is to attach a pressure-cuff to the rinse bag whereby suction may be carried out using a suction-off apparatus. It is also possible to use an infusion pump as an alternative to instillation.

Taurolidine and taurultam solutions may conveniently be applied under pressure, e.g. approx. 10-12 mm Hg, *via* a trocar tube or sleeve by means of a micro-pump. Administered in this way, the solution enters the abdominal cavity as an aerosol, resulting in a more widespread application of the solution to all exposed interabdominal (interior and lateral) surfaces during surgery. Administration of the solution as an aerosol also results in an increased efficacy during pneumoperitoneum with carbon dioxide.

As regards the intravenous infusion of the second portion of solution, it is particularly advantageous to administer Taurolin 2% intravenously through a central catheter as a drop infusion, e.g. at a dosage of 4 x 250 ml per day). If necessary, the drop infusion may be continued for 2 - 3 days following surgery.

### Test Procedure

To prevent intraperitoneal tumour growth and trocar metastases caused by laparoscopic operations, the effect of taurolidine was investigated on the growth of colon carcinoma cells (DHD/L12/TRb) *in vitro,* as well as in rat models. After incubation of the cells with taurolidine there followed an *in vitro* determination of the growth kinetics of the cells. A second experiment followed on rats (n=30) following intraperitoneal application of tumour cells and subsequently the development of a pneumoperitoneum for 30 mins. The rats were randomised into two groups:
I Tumour cells
II Tumour cells + taurolidine

### Results

A significant suppression of tumour growth *in vitro* was observed with taurolidine. *In vivo* the intraperitoneal tumour weight compared to the control group (596 ± 278 mg) was reduced with the instillation of taurolidine (149 ± 247 mg). The development of trocar metastases could be significantly suppressed using taurolidine.

The following non-limiting examples serve to illustrate administration of the first portion of a taurolidine solution through a trocar tube or sleeve in a laparoscopic procedure.

### Example 1

In a typical abdominal procedure, which should not be considered as limiting, a 0.5% taurolidine Ringer solution at body temperature is rinsed through the suction rinse tube under minimal pressure intraoperatively.

According to the extent of surgical invasion, from 100-250 ml 2% taurolidine is instilled at 37°C and allowed to remain in the abdominal cavity on conclusion of the operative procedure.

### Example 2

In a typical abdominal procedure, a taurolidine solution is administered in the form of an aerosol. This may be achieved through the use of a micro-pump which is situated between a gas (e.g. CO₂) supply and the abdominal cavity in which surgery is to be performed. A tube is used to carry the aerosol into a trocar tube or sleeve. The taurolidine solution may be administered continuously as a spray during abdominal surgery, e.g. at a rate of 100 to 200 ml per hour.

## Claims

1. Use of a solution comprising taurolidine, taurultam or a mixture thereof in the manufacture of a medicament for preventing or reducing metastatic growth following the use of trocars during laparoscopic surgery, **characterised in that** said medicament is manufactured for administration (i) in a first portion through a trocar tube or sleeve prior to, during or after said surgery and (ii) in a second portion by intravenous infusion prior to, during or after said surgery.

2. Use as claimed in claim 1 wherein the solution contains from 0.5 to 3% by weight of taurolidine or from 2 to 3% by weight of taurultam.

3. Use as claimed in claim 1 or claim 2 wherein the first portion of said medicament is manufactured for administration using a micro-pump so as to exit said trocar tube or sleeve in the form of an aerosol.

## Revendications

1. Utilisation d'une solution comprenant de la taurolidine, du taurultam ou un mélange de taurolidine et de taurultam pour la fabrication d'un médicament pour prévenir ou réduire une croissance métastasique après utilisation de trocarts au cours d'une procédure chirurgicale laparoscopique, **caractérisé en ce que** ledit médicament est fabriqué pour administration (i) dans une première partie via un tube ou une douille de trocart avant, pendant ou après ladite procédure chirurgicale et (ii) dans une seconde partie par perfusion intraveineuse avant, pendant ou après ladite procédure chirurgicale.

2. Utilisation selon la revendication 1 dans laquelle la solution contient de 0,5 à 3 % en poids de taurolidine ou de 2 à 3 % en poids de taurultam.

3. Utilisation selon la revendication 1 ou la revendication 2 dans laquelle la première partie dudit médicament est fabriquée pour administration par une micro-pompe de manière à sortir dudit tube ou douille de trocart sous forme d'aérosol.

## Patentansprüche

1. Verwendung einer Lösung, die Taurolidin, Taurultam oder ein Gemisch hieraus enthält, bei der Herstellung eines Medikaments zur Verhinderung oder Verringerung des Metastasenwachstums nach dem Einsatz von Trokaren bei der laparoskopischen Chirurgie, **dadurch gekennzeichnet, daß** das Medikament hergestellt wird für die Verabreichung (i) zu einem ersten Teil durch ein Trokarrohr oder eine Trokarhülse vor, während oder nach dem Eingriff und (ii) zu einem zweiten Teil durch intravenöse Infusion vor, während oder nach dem Eingriff.

2. Verwendung nach Anspruch 1, bei der die Lösung 0,5 bis 3 Gew.% Taurolidin oder 2 bis 3 Gew.% Taurultam enthält.

3. Verwendung nach Anspruch 1 oder 2, bei der der erste Teil des Medikaments hergestellt wird für die Verabreichung unter Verwendung einer Mikropumpe, damit er das Trokarrohr oder die Trokarhülse in der Form eines Aerosols verläßt.
